# FASCICULE DE BREVET EUROPEEN

(11) **EP 1 529 493 B1**
(45) Date de publication et mention de la délivrance du brevet: **27.06.2007**
(21) Numéro de dépôt: 04352013.9
(22) Date de dépôt: 04.11.2004
(51) Int. Cl.: A61B 17/72, A61F 2/38

(54) **Orthèse implantable et kit chirurgical pour l'arthrodèse du genou**
Implantierbare Orthese und chirurgischer Satz für eine Arthrodese des Knies
Implantable orthese and surgical kit for the arthrodesis of knee

(30) Priorité: 05.11.2003 FR 0312997
(43) Date de publication de la demande: 11.05.2005
(73) Titulaire: Ceravic, 65500 Vic en Bigorre (FR)
(72) Inventeur: Leonard, Alain, 65500 Caixon (FR)
(74) Mandataire: Morelle, Guy Georges Alain

(56) Documents cités:
- DE-A- 19 722 389
- DE-A- 19 962 324
- FR-A- 2 800 987
- US-A- 4 676 797
- US-A- 5 108 398

## Description

L'invention concerne une orthèse implantable pour réaliser l'arthrodèse du genou.

L'arthrodèse du genou consiste à réunir et solidariser par reconstruction osseuse les extrémités proximales du tibia après résection des épiphyses.

Cette opération est indiquée dans le cas de complications sévères, notamment infectieuses, consécutives à la pose de prothèses articulées, ou suite à des traumatismes importants ou à des exérèses osseuses pour le traitement de tumeurs malignes empêchant la pose de prothèses.

Le principe d'une arthrodèse à l'aide d'un dispositif implantable consiste à implanter dans les parties métaphysaires et médullaires du fémur et du tibia, un matériel d'immobilisation fémoro-tibial permettant le maintien des os immobilisés pendant la reconstruction osseuse périphérique et la prise d'une greffe osseuse intercalaire réalisée.

Ce matériel d'immobilisation implantable consiste traditionnellement en un clou intramédullaire (clou de Juvara) de faible diamètre, typiquement 12 mm, permettant une reconstruction osseuse maximum autour de ce clou, et de longueur importante pour venir s'ancrer dans le canal médullaire du fémur et du tibia. Ces clous posent néanmoins des problèmes de pose, de formes inadaptées aux formes naturelles -notamment aux angles en valgus ou en flexion et à la courbure du fémur-.

Pour pallier ces problèmes, FR-2 800 987 a proposé un dispositif implantable bi-éléments fabriqué sur mesure mais qui permet une intervention chirurgicale limitée en temps, et entraîne un traumatisme post-opératoire moins important qu'un clou intramédullaire. Ce dispositif comprend un implant fémoral à tige intramédullaire fémorale et un implant tibial à tige intramédullaire tibiale, ainsi qu'une pièce de liaison rigide des extrémités proximales de ces deux implants l'un par rapport à l'autre, au droit de l'articulation. Chaque tige intramédullaire comprend un premier tronçon distal de faible diamètre, un deuxième tronçon intermédiaire légèrement tronconique, et un troisième tronçon proximal à section constante de plus fort diamètre. La pièce de liaison rigide est interposée entre les deux implants avec lesquels elle s'emboîte. Elle présente un corps cylindrique doté d'alésages usinés de façon à réaliser les angles de valgus et de flexum, et deux ailettes radiales anti-rotation.

Ce dispositif implantable présente l'inconvénient de devoir être fabriqué sur mesure préalablement à l'intervention pour chaque patient. Or, lors de l'implantation, il arrive que la qualité des os du patient soit beaucoup moins bonne que celle attendue au vu des examens et radiographies préalablement réalisés. En effet, l'arthrodèse est une solution envisagée en général après de multiples interventions, l'échec de solutions prothétiques successives, ou suite à des traumatismes graves du genou. Le site d'implantation a souvent fait l'objet d'une infection ou d'infections multiples ayant détérioré le tissu osseux, notamment au niveau des extrémités proximales des os. Le dispositif implantable préfabriqué sur mesure décrit par FR-2 800 987 ne peut alors pas être mis en place. Le tronçon intermédiaire faiblement conique des tiges qui s'appuie sur le canal médullaire offre un appui insuffisant et n'est pas calé axialement avec une précision suffisante dans l'os. Et malgré le fait que les extrémités proximales des implants soient aussi peu encombrantes que possible dans la direction radiale pour favoriser la reconstruction osseuse périphérique femoro-tibiale, il s'avère que cette reconstruction osseuse est souvent problématique.

En pratique, l'inventeur a déterminé que les échecs rencontrés avec le dispositif implantable connu sus-décrit proviennent en fait d'un calage insuffisant des implants par rapport aux os, d'une mauvaise adaptation dimensionnelle des implants malgré la fabrication sur mesure, et d'un maintien insuffisant des deux os l'un par rapport à l'autre, notamment en torsion.

US-5 108 398 décrit aussi un dispositif implantable pour arthrodèse, de longueur réglable, comprenant une tige fémorale, une tige tibiale et un cylindre vissé de réglage de longueur de la tige fémorale. Là encore, l'inventeur a déterminé que ces implants ne sont pas suffisamment bien calés par rapport aux os et ne réalisent pas un maintien suffisamment important, notamment en torsion, malgré la présence de vis transversales distales.

Il est à noter en outre qu'un dispositif implantable d'arthrodèse est une orthèse qui, contrairement à un implant prothétique, a pour fonction essentielle de maintenir l'ankylose post-opératoire pendant une durée suffisante pour permettre la reconstruction osseuse de la greffe entre le fémur et le tibia (comme les fixateurs externes ou les clous de Juvara antérieurement utilisés). Compte tenu de cette ankylose, il ne peut pas être envisagé de faire supporter l'ensemble des contraintes de la marche à l'implant. Si le dispositif implantable de FR-2 800 987 permet au patient de retrouver une verticalité avant 1a fin de la reconstruction, il n'est pas conçu pour supporter de façon durable l'ensemble des contraintes mécaniques liées à la marche. La nécessité de cette ostéofusion périphérique et les problèmes spécifiques liés à l'ankylose recherchée font que les solutions envisagées pour les prothèses articulaires partielles ou totales destinées à remplacer tout ou partie de l'articulation, en assurant la mobilité et en supportant durablement les contraintes mécaniques de la marche, sont inadaptées et non transposables aux orthèses pour arthrodèse. En particulier, dans une prothèse articulaire, le problème de l'encombrement radial des implants aux extrémités proximales ne se pose pas.

DE-19 722 389 a proposé un dispositif pour arthrodèse formé à la façon d'une prothèse bloquée dont les condyles sont remplacés par des plaques fixées sur des tiges intramédullaires symétriques de révolution, ces plaques venant en appui axial sur les extrémités proximales réséquées des os, les deux plaques étant solidarisées l'une à l'autre par un montage en queue d'aronde. Ce dispositif empêche toute reconstruction osseuse périphérique et est sensé supporter à lui seul durablement toutes les contraintes mécaniques fémoro-tibiales, ce qui est impossible dans le cas de l'ankylose. En effet, les deux os immobilisés génèrent entre eux des contraintes mécaniques notamment en flexion et en torsion de valeurs extrêmement importantes, et en tout cas beaucoup plus importantes que lorsqu'une possibilité de mouvement est préservée. Le dispositif décrit dans ce document est voué à se rompre et en tout cas ne fournit pas un ancrage des implants dans les os qui soit compatible avec le maintien de l'ankylose malgré les structures de contact spécifiques implant/os préconisées par ce document. Il est certain d'observer à court ou moyen terme après l'implantation, un jeu radial entre les implants et les os. L'impossibilité d'utiliser en réalité le dispositif de DE-19 722 389 est donc en pratique une illustration de ce que les solutions envisagées pour les prothèses ne sont pas applicables au cas des arthrodèses.

L'invention vise à pallier ces inconvénients en proposant une orthèse implantable qui assure un maintien amélioré du tibia et du fémur immobilisés, sans risque de dégradation osseuse ultérieure, et tout en permettant et en favorisant une ostéofusion subséquente entre le fémur et le tibia à la périphérie de l'orthèse entre les extrémités réséquées de ces os.

L'invention vise aussi à proposer une telle orthèse qui soit facile à implanter et puisse être préfabriquée en plusieurs tailles et formes standard pouvant convenir à la majorité des applications, sans nécessiter une fabrication sur mesure.

L'invention vise en outre à proposer un kit chirurgical pour la pose d'une orthèse selon l'invention.

L'invention concerne donc une orthèse implantable pour l'arthrodèse du genou comprenant un implant fémoral comportant une tige fémorale adaptée pour pouvoir être introduite dans le canal médullaire du fémur, un implant tibial comportant une tige tibiale adaptée pour pouvoir être introduite dans le canal médullaire du tibia, l'implant fémoral présentant une extrémité proximale de fixation adaptée pour pouvoir coopérer avec une extrémité proximale de fixation conjuguée de l'implant tibial de façon à former une fixation rigide des deux implants l'un par rapport à l'autre autorisant une reconstruction osseuse périphérique entre le tibia et le fémur et autour de ces extrémités proximales de fixation ,
caractérisée en ce que :
- l'implant tibial présente une tête d'ancrage tibiale proximale de forme non symétrique de révolution et évasée dans un plan frontal à partir de la tige tibiale, cette tête d'ancrage tibiale étant adaptée pour pouvoir être introduite dans une cavité évasée de formes conjuguées ménagée dans l'extrémité proximale du tibia, cette tête d'ancrage tibiale présentant une extrémité proximale formant la portion la plus large de l'implant tibial et constituant ladite extrémité proximale de fixation ,
- l'implant fémoral présente une tête d'ancrage fémorale proximale de forme non symétrique de révolution et évasée dans un plan frontal à partir de la tige fémorale, cette tête d'ancrage fémorale étant adaptée pour pouvoir être introduite dans une cavité évasée de formes conjuguées ménagée dans l'extrémité proximale du fémur, cette tête d'ancrage fémorale présentant une extrémité proximale formant la portion la plus large de l'implant fémoral et constituant ladite extrémité proximale de fixation.

L'inventeur a en effet constaté avec surprise que l'adoption de têtes d'ancrage proximales évasées en largeur et dissymétriques de révolution, non seulement ne nuit pas en fait à la reconstruction osseuse périphérique, mais au contraire, en améliorant considérablement la qualité de l'immobilisation relative du tibia par rapport au fémur, favorise cette reconstruction osseuse, au bénéfice d'une plus grande qualité de l'arthrodèse et de résultats améliorés, même dans les cas les plus difficiles. En effet, les têtes d'ancrage évasées viennent combler la partie interne des extrémités proximales des os généralement dégradée suite aux échecs prothétiques antérieurs et/ou aux infections et/ou aux traumatismes et/ou pathologies. Elles imposent en outre un tassement en compression des portions de tissu osseux dégradées. Néanmoins, elles préservent les parties périphériques épiphysaires et métaphysaires, généralement saines, à partir desquelles une ostéofusion se produit sur la greffe osseuse intercalaire. Il est à noter à cet égard que l'on sait que si l'os tend à se consolider sous l'effet d'une sollicitation répétée en charge générale, il se dégénère par contre rapidement en cas d'attaque localisée, par exemple sous l'effet d'un jeu ou d'une concentration de contraintes. En évitant ces phénomènes localisés de jeu ou de concentration de contraintes, malgré un encombrement invasif plus important dans l'os, l'orthèse selon l'invention renforce en fait la rigidité et la solidité de l'arthrodèse obtenue.

Avantageusement et selon l'invention, la tête d'ancrage tibiale est formée d'une pièce distincte de la tige tibiale, et la tête d'ancrage tibiale et la tige tibiale présentent des extrémités d'assemblage rigide adaptées pour permettre leur assemblage et leur fixation rigide dans le prolongement l'une de l'autre avant implantation. De même, avantageusement et selon l'invention, la tête d'ancrage fémorale est formée d'une pièce distincte de la tige fémorale, et la tête d'ancrage fémorale et la tige fémorale présentent des extrémités d'assemblage rigide adaptées pour permettre leur assemblage et leur fixation rigide dans le prolongement l'une de l'autre avant implantation.

De la sorte, une orthèse selon l'invention présente une grande modularité. Il est en particulier possible de prévoir un assortiment de têtes d'ancrage de formes et dimensions variées (par exemple 2 ou 3 tiges tibiales et entre 4 et 8 tiges fémorales). Avec de tels assortiments représentant un nombre de pièces distinctes relativement faible, on arrive à proposer un grand nombre (par exemple entre 72 et 2400) de combinaisons différentes et donc d'orthèses, permettant de répondre à la majorité des cas pratiques pouvant être rencontrés, sans nécessiter de fabrication sur mesure.

Avantageusement et selon l'invention, lesdites extrémités d'assemblage rigide réalisent un assemblage par emboîtement relatif (de la tête et de la tige) et l'orthèse comprend des moyens de serrage et de blocage relatif de ces extrémités l'une par rapport à l'autre. Cet assemblage par emboîtement peut être de type cylindrique ou tronconique.

De même, avantageusement et selon l'invention, les extrémités proximales de fixation de la tête d'ancrage tibiale et de la tête d'ancrage fémorale sont adaptées pour pouvoir être assemblées et fixées rigidement l'une par rapport à l'autre par emboîtement relatif après implantation de l'implant tibial et de l'implant fémoral. Avantageusement et selon l'invention, l'orthèse comprend des moyens de serrage et blocage relatif des extrémités proximales de fixation l'une par rapport à l'autre, notamment sous la forme d'une vis latérale adaptée pour s'étendre dans des alésages ménagés dans les têtes d'ancrage tibiale et fémorale et orientés dans un plan frontal, l'un de ces alésages étant taraudé. Là encore, l'assemblage peut être de type cylindrique ou tronconique. La vis est avantageusement évasée de façon à entraîner un serrage relatif axial des têtes d'ancrages au fur et à mesure de son serrage.

Avantageusement et selon l'invention au moins l'une des têtes d'ancrage -notamment les deux têtes d'ancrage- est(sont) de forme évasée dans un plan sagittal à partir de la tige tibiale ou fémorale correspondante, avec un angle d'évasement sagittal inférieur à l'angle d'évasement frontal de sorte que l'extrémité proximale de fixation présente une profondeur sagittale inférieure à sa largeur frontale.

Avantageusement et selon l'invention au moins l'une des têtes d'ancrage -notamment les deux têtes d'ancrage- comprend(comprennent) deux méplats frontaux antérieur et postérieur s'étendant avec un angle d'évasement sagittal l'un par rapport à l'autre.

Avantageusement et selon l'invention au moins l'une des têtes d'ancrage -notamment les deux têtes d'ancrage- comprend(comprennent) au moins une rainure longitudinale.

Avantageusement et selon l'invention au moins l'une des têtes d'ancrage -notamment les deux têtes d'ancrage- comprend(comprennent) quatre rainures d'angle longitudinales à section transversale courbe le long des bords longitudinaux de chaque méplat.

L'invention s'étend à un kit chirurgical pour l'arthrodèse du genou, caractérisé en ce qu'il comprend une pluralité d'orthèses implantables préfabriquées selon l'invention, de dimensions et/ou formes différentes. Avantageusement et selon l'invention, ce kit comprend une pluralité de têtes d'ancrage tibiales, une pluralité de têtes d'ancrage fémorales, une pluralité de tiges tibiales, une pluralité de tiges fémorales, de dimensions et/ou formes différentes.

En outre, avantageusement et selon l'invention, le kit comprend des calques représentant chaque pièce d'orthèse qu'il contient, adaptés pour permettre le choix de l'orthèse implantable préfabriquée par superposition avec une radiographie. Egalement, un kit selon l'invention comprend une pluralité d'ancillaires adaptés chacun à la pose d'une des pièces des orthèses implantables préfabriquées.

L'invention s'étend à une orthèse implantable pour arthrodèse et à un kit chirurgical caractérisés en combinaison par tout ou partie des caractéristiques mentionnées ci-dessus ou ci-après.

D'autres buts, avantages et caractéristiques de l'invention apparaissent à la lecture de la description suivante qui se réfère aux figures annexées dans lesquelles :
- la figure 1 est une vue schématique frontale d'une orthèse selon l'invention après implantation,
- la figure 2 est une vue schématique sagittale d'une orthèse,

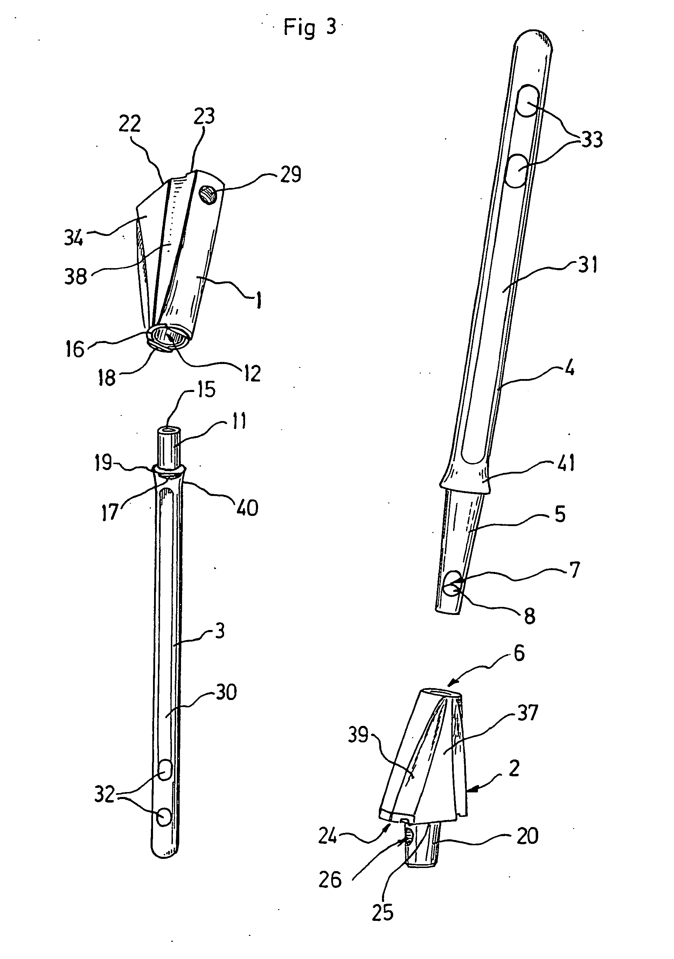

- la figure 3 est une vue schématique en perspective éclatée d'une orthèse selon l'invention,

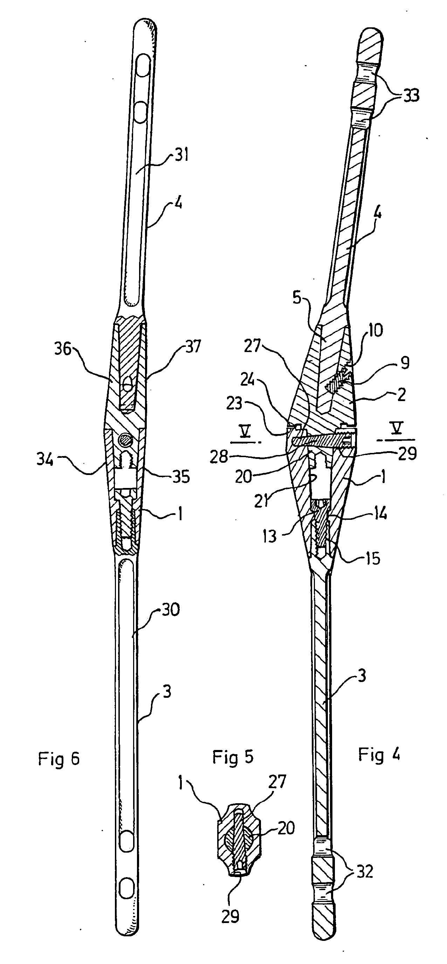

- la figure 4 est une vue schématique en coupe frontale d'une orthèse selon l'invention,
- la figure 5 est une vue schématique selon la ligne V-V de la figure 4,
- la figure 6 est une vue schématique en coupe sagittale d'une orthèse selon l'invention.

L'orthèse selon l'invention représentée sur les figures comprend un tête d'ancrage tibiale proximale 1, une tête d'ancrage fémorale proximale 2, une tige intramédullaire tibiale distale 3, et une tige intramédullaire fémorale distale 4. Elle est ainsi composée de quatre pièces principales, ainsi que de trois vis 9, 13, 28 de blocage et serrage de ces pièces les unes aux autres.

Pour éviter toute erreur au montage, les organes d'assemblage de la tige tibiale 3 sur la tête d'ancrage tibiale 1 ne sont pas compatibles avec les organes d'assemblage de la tige fémorale sur la tête d'ancrage fémorale 2.

Pour l'assemblage de la tête d'ancrage fémorale 2 sur la tige intramédullaire fémorale 4, cette dernière est dotée d'un cône d'assemblage 5 mâle adapté pour pouvoir être introduit dans un cône femelle 6 conjugué de la tête d'ancrage fémorale 2. L'extrémité du cône mâle 5 présente un évidement 7 formant une portée d'appui 8 pour l'extrémité libre d'une vis de serrage 9 introduite dans un alésage taraudé 10 de la tête d'ancrage fémorale 2. L'axe de cet alésage taraudé 10 est incliné par rapport à l'axe des cônes 5, 6 de telle sorte que lorsque la vis 9 est serrée, son extrémité libre vient en appui contre la portée d'appui 8 en serrant axialement les deux cônes 5, 6 l'un dans l'autre.

La tige intramédullaire tibiale 3 comprend un cylindre mâle 11 à son extrémité proximale adapté pour pouvoir pénétrer dans un cylindre femelle 12 ménagé dans la tête d'ancrage tibiale 1, pour l'assemblage de la tige tibiale 3 sur cette tête d'ancrage tibiale 1. Une vis de serrage axial 13 peut être introduite axialement à travers la tête tibiale 1 de façon à prendre appui sur un épaulement 14 de cette dernière et pour que son extrémité filetée puisse être introduite dans un alésage taraudé 15 du cylindre 11 d'assemblage de la tige tibiale 3. Lorsque la vis 13 est serrée, la tige tibiale 3 se trouve assemblée serrée sur la tête d'ancrage tibiale 1. En outre, la tête tibiale 1 et la tige tibiale 3 présentent des échancrures 16, respectivement 17, et des saillies conjuguées 18, respectivement 19, réalisant un blocage en rotation axiale de la tête tibiale 1 par rapport à la tige tibiale 3.

La tête d'ancrage fémorale 2 comprend un tronc de cône mâle 20 adapté pour pouvoir être introduit dans un tronc de cône femelle 21 conjugué de la tête d'ancrage tibiale 1, en vue d'un assemblage de type conique doux entre ces deux têtes 1,2.

L'extrémité proximale de fixation 22 de la tête d'ancrage tibiale 1 présente avantageusement une saillie latérale axiale 23 destinée à se loger dans un renfoncement conjugué 24 de l'extrémité proximale de fixation 25 de la tête fémorale 2. Cette saillie 23 et ce renfoncement 24 constituent ainsi un détrompeur empêchant tout montage inversé.

Le tronc de cône 20 de la tête d'ancrage fémorale 2 comprend un alésage 26 transversal radial évasé, notamment sensiblement tronconique, adapté pour recevoir l'extrémité libre. Cette vis 28 est pointue, notamment sensiblement tronconique 27 d'une vis 28 de blocage et serrage des deux têtes 1, 2 assemblées. Cette vis 28 est serrée dans un alésage 29 taraudé transversal radial de la tête d'ancrage tibial 1. Etant pointue, c'est-à-dire évasée à l'opposé de son extrémité libre 27, son vissage entraîne le serrage axial des deux têtes d'ancrage 1, 2 au fur et à mesure qu'elle est vissée dans l'alésage 26.

L'alésage 29 taraudé est positionné de façon à venir en regard de l'alésage 26 du tronc de cône 20 de la tête d'ancrage fémorale 2 lorsque cette dernière est assemblée sur la tête d'ancrage tibiale1. L'alésage 29 taraudé débouche extérieurement latéralement, c'est-à-dire sur le côté de la tête d'ancrage tibiale 1, de sorte qu'il est accessible par le côté de l'orthèse lors de l'implantation. Il est disposé à proximité de l'extrémité proximale de fixation 22 de la tête d'ancrage tibiale 1, qui s'étend en général, après implantation de l'implant tibial, en saillie hors du tibia 43, c'est-à-dire au-delà de la partie réséquée de l'extrémité proximale du tibia 43, De la sorte, la vis 28 est accessible pour le serrage après implantation de l'implant tibial 1, 3.

La tête d'ancrage fémorale 2 est adaptée pour réaliser un angle de valgus et un angle de flexion, c'est-à-dire que les axes respectifs de son tronc de cône 20 d'assemblage sur la tête d'ancrage tibiale 1 et de son tronc de cône femelle 6 d'assemblage à la tige fémorale 4 sont orientés de façon à réaliser ces angles.

La tige fémorale 4 peut également présenter une courbure conforme à la courbure naturelle du fémur 44. Chacune des tiges 3, 4 est dotée de rayures longitudinales latérales 30, respectivement 31, ainsi que d'alésages traversants transversaux distaux 32, respectivement 33, permettant leur fixation distale par des vis transversales de façon connue en soi.

Les têtes d'ancrage tibiale 1 et fémorale 2 présentent un angle d'évasement frontal au sommet (angle au sommet du trapèze qui circonscrit la section axiale frontale de la tête 1, 2) qui est compris entre 20 degrés et 45 degrés, et plus particulièrement, dans les modes de réalisation préférés, entre 22 degrés et 37 degrés.

Chaque tête d'ancrage 1, 2 est en effet évasée depuis son extrémité d'assemblage à la tige 3, 4 correspondante, jusqu'à son extrémité proximale de fixation 22, 25 respectivement, qui forme la portion la plus large de cette tête 1, 2 et donc de l'orthèse. Les têtes d'ancrage 1, 2 présentent à leur extrémité proximale de fixation 22, 25, une largeur frontale comprise entre 20mm et 40mm -notamment de l'ordre de 30mm-.

Les têtes d'ancrage 1, 2 sont également de formes évasées dans le plan sagittal à partir de la tige intramédullaire correspondante 1, 4 avec un angle d'évasement sagittal inférieur à l'angle d'évasement frontal, de sorte que l'extrémité proximale de fixation 22, 25 présente une profondeur sagittale inférieure à sa largeur frontale. L'angle d'évasement sagittal au sommet (angle au sommet du trapèze qui circonscrit la section axiale sagittale de la tête 1, 2) est avantageusement compris entre 10 degrés et 25 degrés, et plus particulièrement dans les modes de réalisation préférés, entre 13 degrés et 23 degrés. Les têtes d'ancrage 1, 2 présentent à leur extrémité proximale de fixation 22, 25 une profondeur sagittale qui peut être comprise entre 15mm et 25mm -notamment de l'ordre de 18mm-.

Les têtes d'ancrage 1, 2 comprennent chacune deux méplats frontaux antérieur 34, 36 et postérieur 35, 37 respectivement, s'étendant avec un angle d'évasement sagittal l'un par rapport à l'autre. En variante non représentée, ces méplats peuvent être plus ou moins gauches, concaves ou convexes en section axiale ou transversale.

En outre, chacune de ces têtes d'ancrage 1 comprend quatre rainures d'angle longitudinales 38, respectivement 39, à section transversale courbe concave, notamment circulaire, le long des bords longitudinaux de chaque méplat 34, 35, 36, 37. Ces rainures d'angle longitudinales 38, 39 favorisent l'insertion de têtes d'ancrage 1, 2 dans l'os 43, 44, 45 en renforçant leur blocage en torsion.

De préférence, comme représenté, chaque tête d'ancrage 1, 2 présente une section dans un plan frontal axial qui est trapézoïdale ou sensiblement trapézoïdale. Chaque tête d'ancrage 1, 2 peut être ainsi formée à partir d'une pièce tronconique ou sensiblement tronconique sur laquelle on a ménagé les méplats 34, 36, 35, 37 et les rainures d'angle 38, 39. Néanmoins d'autres formes évasées, par exemple concaves ou convexes, sont possibles.

La section droite transversale non symétrique de révolution des tiges tibiale et fémorale 3, 4, ainsi que les éventuelles vis transversales distales renforcent le blocage en torsion de ces tiges 3, 4 par rapport à l'os 43, 44. Chaque tige tibiale et fémorale 3, 4 présentent de préférence une section droite transversale de dimensions au moins sensiblement constantes le long de cette tige 3, 4, à l'exception éventuellement d'un congé 40, 41 de raccordement à la tête d'ancrage correspondante 1, 2. Ce congé 40, 41 n'existe pas si le diamètre de la tige intramédullaire est le même que celui de l'extrémité distale de la tête d'ancrage 1, 2 correspondante.

Le tableau 1 ci-après donne un exemple de différents dimensionnements possibles en millimètres des différentes pièces constituant une orthèse selon l'invention.

| | | JAMBE ASSOCIÉE | DIAMETRE | LONGUEUR | COURBURE |
|---|---|---|---|---|---|
| IMPLANT TIBIAL | TIGE | Indifférent | 9.5 | 165 | |
| | | Indifférent | 11.5 | 165 | |
| | TÊTE | Indifférent | | 45 | |
| | | Indifférent | | 50 | |
| | | Indifférent | | 55 | |
| | | Indifférent | | 60 | |
| | | Indifférent | | 65 | |
| IMPLANT FEMORAL | TIGE | Indifférent | 10 | 130 | 900 |
| | | Indifférent | 12 | 130 | 900 |
| | | Indifférent | 14 | 130 | 900 |
| | | Indifférent | 10 | 160 | 900 |
| | | Indifférent | 10 | 160 | 1050 |
| | | Indifférent | 12 | 160 | 900 |
| | | Indifférent | 12 | 160 | 1050 |
| | | Indifférent | 14 | 160 | 900 |
| | | Indifférent | 14 | 160 | 1050 |
| | TÊTE | Gauche | | 45 | |
| | | Gauche | | 60 | |
| | | Droite | | 45 | |
| | | Droite | | 60 | |

Toutes les pièces correspondant à cette gamme peuvent être rassemblées dans un conteneur formant un kit chirurgical, le chirurgien choisissant les pièces de dimensions appropriées lors même de l'opération en fonction du besoin. La longueur des pièces est déterminée pour obtenir une stabilisation du membre en prenant en compte un raccourcissement de 2 cm. Les diamètres des tiges 3, 4 et les dimensions des têtes 1, 2 sont adaptés en fonction de la morphologie osseuse et de la qualité du tissu osseux.

En variante, rien n'empêche également de prévoir plusieurs dimensions latérales et sagittales pour les têtes d'ancrage.

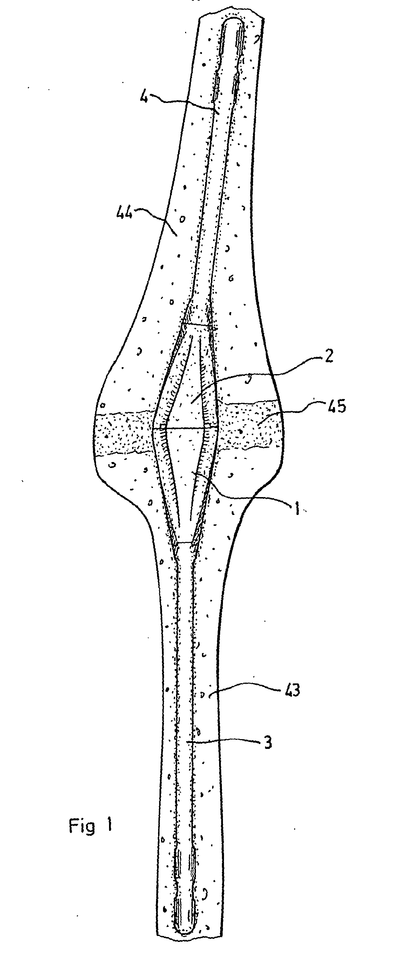

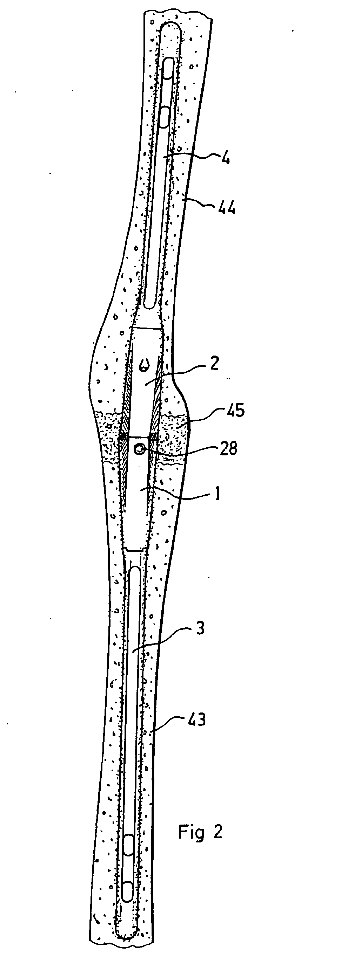

Comme on le voit figures 1 et 2, la longueur des implants tibial 1, 3 et fémoral 2, 4 assemblés rigidement l'un à l'autre par les extrémités de fixation 22, 25 des têtes d'ancrage 1, 2, détermine la largeur totale fémorale de la jambe. Entre l'extrémité proximale réséquée du tibia 43 et celle du fémur 44, on peut insérer une greffe osseuse 45 et/ou tout matériau ostéoconducteur, autour des parties des têtes d'ancrage 1, 2 saillant hors du tibia 43 et du fémur 44. L'orthèse maintient les os 43, 44 immobilisés pendant la prise de la greffe 45 qui, ensuite, assure le soutien et l'ankylose.

Le kit chirurgical selon l'invention comprend également avantageusement des calques représentant chacune de ces pièces en coupe frontale et en coupe sagittale, qui peuvent être superposés à des radiographies du patient pour faciliter le choix des pièces avant même l'opération chirurgicale. Egalement, le kit chirurgical selon l'invention comprend le matériel ancillaire permettant la préparation des os et la mise en place de l'orthèse. Ce matériel ancillaire peut comprendre notamment des râpes de formes similaires à celles des têtes 1, 2 destinées à être manoeuvrées uniquement axialement à l'intérieur des os pour réaliser les cavités de réception de ces têtes d'ancrage 1, 2. Il peut comprendre également des guides de perçage dans les canaux médullaires et des étaux de viseur.

La surface des têtes d'ancrage 1, 2 en contact avec l'os ainsi que celle d'ailleurs des tiges 3, 4 peut être laissée lisse ou au contraire revêtue d'un revêtement facilitant l'ostéo-intégration, notamment un revêtement d'hydroxyapatite ou autre.

L'assemblage conique des deux têtes d'ancrage 1, 2 l'une sur l'autre est adapté pour permettre l'emboîtement des troncs de cône 20, 21 l'un dans l'autre par basculement à partir de la position fléchie à 90 degrés du tibia par rapport au fémur, position dans laquelle on réalise l'implantation de l'implant tibial 1, 3 et de l'implant fémoral 2, 4.

La tête d'ancrage tibiale 1 est assemblée et serrée sur la tige tibiale 3 avant son implantation dans le tibia. De même, la tête d'ancrage fémorale 2 est assemblée serrée sur la tige fémorale 4 avant implantation dans le fémur. Après assemblage et serrage des deux têtes 1, 2 par la vis 28, les deux os sont maintenus immobilisés l'un par rapport à l'autre avec les angles de valgus et de flexus appropriés définis par la tête d'ancrage fémorale 2.

Malgré l'encombrement relativement important des têtes d'ancrage 1, 2 aux extrémités proximales des os, on constate en pratique une très bonne reconstruction osseuse et une fusion de bonne qualité entre le tibia et le fémur aboutissant à bref délai à une arthrodèse également de bonne qualité.

Le patient peut être verticalisé peu de temps (typiquement 24h) après l'opération chirurgicale compte tenu de la fixation excellente formée par l'orthèse selon l'invention.

## Revendications

1. - Orthèse implantable pour l'arthrodèse du genou comprenant un implant fémoral (2, 4) comportant une tige fémorale (4) adaptée pour pouvoir être introduite dans le canal médullaire du fémur, un implant tibial (1, 3) comportant une tige tibiale (3) adaptée pour pouvoir être introduite dans le canal médullaire du tibia, l'implant fémoral (2, 4) présentant une extrémité proximale de fixation (25) adaptée pour pouvoir coopérer avec une extrémité proximale de fixation (22) conjuguée de l'implant tibial (1, 3) de façon à former une fixation rigide des deux implants l'un par rapport à l'autre autorisant une reconstruction osseuse périphérique entre le tibia et le fémur et autour de ces extrémités proximales de fixation (22, 25), **caractérisée en ce que :**
- l'implant tibial (1, 3) présente une tête d'ancrage tibiale (1) proximale de forme non symétrique de révolution et évasée dans un plan frontal à partir de la tige tibiale (3), cette tête d'ancrage tibiale (1) étant adaptée pour pouvoir être introduite dans une cavité évasée de formes conjuguées ménagée dans l'extrémité proximale du tibia, cette tête d'ancrage tibiale (1) présentant une extrémité proximale formant la portion la plus large de l'implant tibial et constituant ladite extrémité proximale de fixation (22), et
- l'implant fémoral (2, 4) présente une tête d'ancrage fémorale (2) proximale de forme non symétrique de révolution et évasée dans un plan frontal à partir de la tige fémorale (4), cette tête d'ancrage fémorale (2) étant adaptée pour pouvoir être introduite dans une cavité évasée de formes conjuguées ménagée dans l'extrémité proximale du fémur, cette tête d'ancrage fémorale (2) présentant une extrémité proximale formant la portion la plus large de l'implant fémoral et constituant ladite extrémité proximale de fixation (25).

2. - Orthèse selon la revendication 1, **caractérisée en ce que** la tête d'ancrage tibiale (1) est formée d'une pièce distincte de la tige tibiale (3), la tête d'ancrage tibiale (1) et la tige tibiale (3) présentant des extrémités (11, 12) d'assemblage rigide adaptées pour permettre leur assemblage et leur fixation rigide dans le prolongement l'une de l'autre avant implantation.

3. - Orthèse selon la revendication 2, **caractérisée en ce que** les extrémités (11, 12) d'assemblage rigide réalisent un assemblage par emboîtement relatif, et **en ce qu'**elle comprend des moyens (13) de serrage et de blocage relatif de ces extrémités (11, 12) l'une par rapport à l'autre.

4. - Orthèse selon l'une des revendications 1 à 3, **caractérisée en ce que** la tête d'ancrage fémorale (2) est formée d'une pièce distincte de la tige fémorale (4), la tête d'ancrage fémorale (2) et la tige fémorale (4) présentant des extrémités (5, 6) d'assemblage rigide adaptées pour permettre leur assemblage et leur fixation rigide dans le prolongement l'une de l'autre avant implantation.

5. - Orthèse selon la revendication 4, **caractérisée en ce que** les extrémités (5, 6) d'assemblage rigide réalisent un assemblage par emboîtement relatif, et **en ce qu'**elle comprend des moyens (9) de serrage et de blocage relatif de ces extrémités (11, 12) l'une par rapport à l'autre.

6. - Orthèse selon l'une des revendications 1 à 5, **caractérisée en ce que** les têtes d'ancrage tibiale (1) et fémorale (2) présentent un angle d'évasement frontal au sommet compris entre 20 degrés et 45 degrés.

7. - Orthèse selon l'une des revendications 1 à 6, **caractérisée en ce qu**'au moins l'une des têtes d'ancrage (1, 2) est de forme évasée dans un plan sagittal à partir de la tige tibiale (3) ou fémorale (4) correspondante, avec un angle d'évasement sagittal inférieur à l'angle d'évasement frontal de sorte que l'extrémité proximale de fixation (22, 25) présente une profondeur sagittale inférieure à sa largeur frontale.

8. - Orthèse selon la revendication 7, **caractérisée en ce que** l'angle d'évasement sagittal au sommet est compris entre 10 degrés et 25 degrés.

9. - Orthèse selon l'une des revendications 1 à 8, **caractérisée en ce que** les têtes d'ancrage (1, 2) présentent à leur extrémité proximale de fixation (22, 25), une largeur frontale comprise entre 20mm et 40mm -notamment de l'ordre de 30mm-.

10. - Orthèse selon l'une des revendications 1 à 9, **caractérisée en ce que** les têtes d'ancrage (1, 2) présentent à leur extrémité proximale de fixation (22, 25), une profondeur sagittale comprise entre 15mm et 25mm -notamment de l'ordre de 18mm-.

11. - Orthèse selon l'une des revendications 1 à 10, **caractérisée en ce qu**'au moins l'une des têtes d'ancrage comprend deux méplats frontaux antérieur (34, 36) et postérieur (35, 37) s'étendant avec un angle d'évasement sagittal l'un par rapport à l'autre.

12. - Orthèse selon l'une des revendications 1 à 11, **caractérisée en ce qu'**au moins l'une des têtes d'ancrage (1, 2) comprend au moins une rainure longitudinale (38, 39).

13. - Orthèse selon l'une des revendications 11 et 12, **caractérisée en ce qu**'au moins l'une des têtes d'ancrage (1, 2) comprend quatre rainures d'angle longitudinales (38, 39) à section transversale courbe le long des bords longitudinaux de chaque méplat (34, 35, 36, 37).

14. - Orthèse selon l'une des revendications 1 à 13, **caractérisée en ce que** les extrémités proximales de fixation (22, 25) de la tête d'ancrage tibiale (1) et de la tête d'ancrage fémorale (2) sont adaptées pour pouvoir être assemblées et fixées rigidement l'une par rapport à l'autre par emboîtement relatif après implantation de l'implant tibial et de l'implant fémoral.

15. - Orthèse selon la revendication 14, **caractérisée en ce qu**'elle comprend des moyens (26, 28) de serrage et blocage relatif des extrémités proximales de fixation (22, 25) l'une par rapport à l'autre.

16. - Orthèse selon la revendication 15, **caractérisée en ce que** ces moyens (26, 28) de serrage et blocage relatif comprennent une vis (28) latérale adaptée pour s'étendre dans des alésages (26, 29) ménagés dans les têtes d'ancrage tibiale (1) et fémorale (2) orientés dans un plan frontal, l'un (29) de ces alésages étant taraudé.

17. - Orthèse selon la revendication 16, **caractérisée en ce que** la vis latérale (28) présente une extrémité (27) évasée de façon à entraîner un serrage relatif axial des têtes d'ancrage (1, 2) au fur et à mesure de son vissage.

18. - Orthèse selon l'une des revendications 1 à 17, **caractérisée en ce que** l'une (2) des têtes d'ancrage (1, 2) comprend un tronc de cône mâle (20) d'assemblage adapté pour pouvoir être introduit dans un tronc de cône femelle (21) d'assemblage conjugué de l'autre (1) tête d'ancrage, de sorte que leur assemblage relatif est de type conique.

19. - Orthèse selon l'une des revendications 1 à 18, **caractérisée en ce que** les tiges tibiale (3) et fémorale (4) présentent une section droite transversale non symétrique de révolution.

20. - Orthèse selon l'une des revendications 1 à 19, **caractérisée en ce que** les tiges tibiale (3) et fémorale (4) présentent une section droite transversale de dimensions au moins sensiblement constantes le long de la tige.

21. - Kit chirurgical pour l'arthrodèse du genou, **caractérisé en ce qu**'il comprend une pluralité d'orthèses implantables préfabriquées selon l'une des revendications 1 à 20, de dimensions et/ou formes différentes.

22. - Kit chirurgical selon la revendication 21, **caractérisé en ce qu**'il comprend une pluralité de têtes d'ancrage tibiales (1), une pluralité de têtes d'ancrage fémorales (2), une pluralité de tiges tibiales (3), une pluralité de tiges fémorales (4), de dimensions et/ou formes différentes.

23. - Kit chirurgical selon l'une des revendications 21 ou 22, **caractérisé en ce qu**'il comprend des calques représentant chaque pièce d'orthèse qu'il contient, adaptés pour permettre le choix de l'orthèse implantable préfabriquée par superposition avec une radiographie.

24. - Kit chirurgical selon l'une des revendications 21 à 23, **caractérisé en ce qu**'il comprend une pluralité d'ancillaires adaptés chacun à la pose d'une des pièces des orthèses implantables préfabriquées.

## Claims

1. An implantable orthesis for knee arthrodesis comprising a femoral implant (2, 4) containing a femoral rod (4) adapted to be able to be introduced into the medullary canal of the femur, a tibial implant (1, 3) comprising a tibial rod (3) adapted to be able to be introduced into the medullary canal of the tibia, the femoral implant (2, 4) having a proximal fixing end (25) adapted to be able to cooperate with a conjugate proximal fixing end (22) of the tibial implant (1, 3) so as to form a rigid fixing of the two implants relative to one another allowing peripheral bone reconstruction between the tibia and the femur and around these proximal fixing ends (22, 25), **characterized in that**:
- the tibial implant (1, 3) has a proximal tibial anchoring head (1) of a shape which is without symmetry of revolution and splayed in a front plane from the tibial rod (3), this tibial anchoring head (1) being adapted to be able to be introduced into a splayed cavity of conjugate forms made in the proximal end of the tibia, this tibial anchoring head (1) having a proximal end forming the widest portion of the tibial implant and constituting said proximal fixing end (22), and
- the femoral implant (2, 4) has a proximal femoral anchoring head (2) of a shape which is without symmetry of revolution and splayed in a front plane from the femoral rod (4), this femoral anchoring head (2) being adapted to be able to be introduced into a splayed cavity of conjugate forms made in the proximal end of the femur, this femoral anchoring head (2) having a proximal end forming the widest portion of the femoral implant and constituting said proximal fixing end (25).

2. An orthesis as claimed in claim 1, **characterized in that** the tibial anchoring head (1) is formed from a specific part of the tibial rod (3), the tibial anchoring head (1) and tibial rod (3) having rigid assembly ends (11, 12) adapted to allow their assembly and their rigid fixing in the extension of one to the other before implantation.

3. An orthesis as claimed in claim 2, **characterized in that** the rigid assembly ends (11, 12) produce an assembly by relative interlocking and **in that** it comprises means (13) for the relative tightening and securing of these ends (11, 12) relative to one another.

4. An orthesis as claimed in one of claims 1 to 3, **characterized in that** the femoral anchoring head (2) is formed from a specific part of the femoral rod (4), the femoral anchoring head (2) and the femoral rod (4) having rigid assembly ends (5, 6) adapted to allow their assembly and their rigid fixing in the extension of one to the other before implantation.

5. An orthesis as claimed in claim 4, **characterized in that** the rigid assembly ends (5, 6) produce an assembly by relative interlocking and **in that** it comprises means (9) for relative tightening and securing of these ends (11, 12) relative to one another.

6. An orthesis as claimed in one of claims 1 to 5, **characterized in that** the tibial (1) and femoral (2) anchoring heads have a front splay angle at the top of between 20 degrees and 45 degrees.

7. An orthesis as claimed in one of claims 1 to 6, **characterized in that** at least one of the anchoring heads (1, 2) is of splayed shape in a sagittal plane from the corresponding tibial (3) or femoral (4) rod, with a sagittal splay angle less than the front splay angle such that the proximal fixing end (22, 25) has a lower sagittal depth than its front width.

8. An orthesis as claimed in claim 7, **characterized in that** the sagittal splay angle at the top is between 10 degrees and 25 degrees.

9. An orthesis as claimed in one of claims 1 to 8, **characterized in that** the anchoring heads (1, 2) have at their proximal fixing end (22, 25) a front width between 20mm and 40mm - specifically 30mm-.

10. An orthesis as claimed in one of claims I to 9, **characterized in that** the anchoring heads (1, 2) have at their proximal fixing end (22, 25) a sagittal depth of between 15mm and 25mm -specifically 18mm-.

11. An orthesis as claimed in one of claims 1 to 10, **characterized in that** at least one of the anchoring heads comprises two front (34, 36) and rear front (35, 37) flats extending with a sagittal splay angle relative to one another.

12. An orthesis as claimed in one of claims 1 to 11, **characterized in that** at least one of the anchoring heads (1, 2) comprises at least one longitudinal groove (38, 39).

13. An orthesis as claimed in one of claims 11 and 12, **characterized in that** at least one of the anchoring heads (1, 2) comprises four angled longitudinal grooves (38, 39) with an arcuate transverse cross-section along the longitudinal edges of each flat (34, 35, 36, 37).

14. An orthesis as claimed in one of claims 1 to 13, **characterized in that** the proximal fixing ends (22, 25) of the tibial anchoring head (1) and the femoral anchoring head (2) are adapted to be able to be assembled and fixed rigidly to one another by relative interlocking after implantation of the tibial implant and the femoral implant.

15. An orthesis as claimed in claim 14, **characterized in that** it comprises means (26, 28) for relative tightening and securing of the proximal fixing ends (22, 25) relative to one another.

16. An orthesis as claimed in claim 15, **characterized in that** those relative tightening and securing means (26, 28) comprise a lateral screw (28) adapted to extend into bores (26, 29) made in the tibial (1) and femoral (2) anchoring heads oriented in a front plane, one (29) of these bores being threaded.

17. An orthesis as claimed in claim 16, **characterized in that** the lateral screw (28) has a splayed end (27) so as to produce relative axial tightening of the anchoring heads (1, 2) as it is screwed in.

18. An orthesis as claimed in one of claims 1 to 17, **characterized in that** one (2) of the anchoring heads (1, 2) comprises a male cone trunk (20) of adapted assembly to be able to be introduced into a female cone trunk (21) of conjugate assembly of the other anchoring head (1), such that their relative assembly is of conical type.

19. An orthesis as claimed in one of claims 1 to 18, **characterized in that** the tibial (3) and femoral (4) rods have a right transverse section which is without symmetry in revolution.

20. An orthesis as claimed in one of claims 1 to 19, **characterized in that** the tibial (3) and femoral (4) rods have a right transverse section of at least substantially constant size along the rod.

21. A surgical kit for knee arthrodesis, **characterized in that** it comprises a plurality of prefabricated implantable ortheses according to one of claims 1 to 20, of different sizes and/or shapes.

22. A surgical kit as claimed in claim 21, **characterized in that** it comprises a plurality of tibial anchoring heads (1), a plurality of femoral anchoring heads (2), a plurality of tibial rods (3), a plurality of femoral rods (4), of different sizes and/or shapes.

23. A surgical kit as claimed in one of claims 21 or 22, **characterized in that** it comprises templates representing each part of the orthesis that it contains, adapted to allow the selection of the prefabricated implantable orthesis by superimposing with an x-ray.

24. A surgical kit as claimed in one of claims 21 to 23, **characterized in that** it comprises a plurality of ancillary parts each adapted to the fitting of one of the prefabricated implantable ortheses.

## Patentansprüche

1. Implantierbare Orthese für die Kniearthrodese, mit einem Femur-Implantat (2, 4), das einen femoralen Schaft (4) aufweist, der dazu angepasst ist, in den Knochenmarkkanal des Oberschenkelknochens eingeführt zu werden, einem Tibia-Implantat (1, 3), das einen tibialen Schaft (3) aufweist, der dazu angepast ist, in den Knochenmarkkanal des Schienbeins eingeführt zu werden, wobei das Femur-Implantat (2, 4) ein proximales Befestigungsende (25) aufweist, das dazu angepasst ist, mit einem dem Tibia-Implantat (1, 3) zugehörigen proximalen Befestigungsende (22) derart zusammenwirken zu können, dass eine starre Befestigung der beiden Implantate zueinander entsteht, wodurch zwischen Schienbein und Oberschenkelknochen und um die proximalen Befestigungsenden (22, 25) herum eine umlaufende Knochenrekonstruktion ermöglicht wird,
**dadurch gekennzeichnet, dass**:
- das Tibia-Implantat (1, 3) einen nicht rotationssymmetrisch geformten proximalen tibialen Verankerungskopf (1) aufweist, der sich vom tibialen Schaft (3) aus in der Frontalebene verbreitert, wobei dieser tibiale Verankerungskopf (1) dazu angepasst ist, in einen sich verbreiternden Hohlraum mit entsprechender Form eingeführt zu werden, der in das proximale Ende des Schienbeins eingesetzt wurde, wobei dieser tibiale Verankerungskopf (1) ein proximales Ende aufweist, das den größten Abschnitt des Tibia-Implantats bildet und das proximale Befestigungsende (22) darstellt, und
- das Femur-Implantat (2, 4) einen nicht rotationssymmetrisch geformten proximalen femoralen Verankerungskopf (2) aufweist, der sich vom femoralen Schaft (4) aus in der Frontalebene verbreitert, wobei dieser femorale Verankerungskopf (2) dazu angepasst ist, in einen sich verbreiternden Hohlraum mit entsprechender Form eingeführt zu werden, der in das proximale Ende des Oberschenkelknochens eingesetzt wurde,
wobei dieser femorale Verankerungskopf (2) ein proximales Ende aufweist, das den größten Abschnitt des Femur-Implantats bildet und das proximale Befestigungsende (25) darstellt.

2. Orthese nach Anspruch 1, **dadurch gekennzeichnet, dass** der tibiale Verankerungskopf (1) aus einem Teil gebildet ist, der sich vom tibialen Schaft (3) unterscheidet, wobei der tibiale Verankerungskopf (1) und der tibiale Schaft (3) Enden (11, 12) zum starren Zusammenbau aufweisen, die dazu angepasst sind, vor der Implantation in der Verlängerung zueinander zusammengesetzt und starr befestigt werden zu können.

3. Orthese nach Anspruch 2, **dadurch gekennzeichnet, dass** die Enden (11, 12) zum starren Zusammenbau eine Verbindung durch Ineinanderstecken herstellen und dass sie Mittel (13) zum Sichern und Feststellen dieser Enden (11, 12) zueinander aufweist.

4. Orthese nach einem der Ansprüche 1 bis 3, **dadurch gekennzeichnet, dass** der femorale Verankerungskopf (2) aus einem Teil gebildet ist, der sich vom femoralen Schaft (4) unterscheidet, wobei der femorale Verankerungskopf (2) und der femorale Schaft (4) Enden (5, 6) zum starren Zusammenbau aufweisen, die dazu angepasst sind, vor der Implantation in der Verlängerung zueinander zusammengesetzt und starr befestigt werden zu können.

5. Orthese nach Anspruch 4, **dadurch gekennzeichnet, dass** die Enden (5, 6) zum starren Zusammenbau eine Verbindung durch Ineinanderstecken herstellen und dass sie Mittel (9) zum Sichern und Feststellen dieser Enden (11, 12) zueinander aufweist.

6. Orthese nach einem der Ansprüche 1 bis 5, **dadurch gekennzeichnet, dass** der tibiale (1) und der femorale (2) Verankerungskopf in der Frontalebene an der Spitze einen Aufweitungswinkel von zwischen 20 Grad und 45 Grad aufweisen.

7. Orthese nach einem der Ansprüche 1 bis 6, **dadurch gekennzeichnet, dass** mindestens einer der Verankerungsköpfe (1, 2) in der Sagittalebene vom entsprechenden tibialen (3) oder femoralen Schaft (4) aus eine sich verbreiternde Form aufweist, mit einem Aufweitungswinkel in der Sagittalebene, der kleiner ist als der Aufweitungswinkel in der Frontalebene, sodass die Tiefe des proximalen Befestigungsendes (22, 25) in der Sagittalebene geringer ist als dessen Breite in der Frontalebene.

8. Orthese nach Anspruch 7, **dadurch gekennzeichnet, dass** der Aufweitungswinkel in der Sagittalebene an der Spitze zwischen 10 Grad und 25 Grad beträgt.

9. Orthese nach einem der Ansprüche 1 bis 8, **dadurch gekennzeichnet, dass** die Verankerungsköpfe (1, 2) an ihrem proximalen Befestigungsende (22, 25) eine Breite in der Frontalebene zwischen 20 mm und 40 mm aufweisen, insbesondere in der Größenordnung von 30 mm.

10. Orthese nach einem der Ansprüche 1 bis 9, **dadurch gekennzeichnet, dass** die Verankerungsköpfe (1, 2) an ihrem proximalen Befestigungsende (22, 25) eine Tiefe in der Sagittalebene zwischen 15 mm und 25 mm aufweisen, insbesondere in der Größenordnung von 18 mm.

11. Orthese nach einem der Ansprüche 1 bis 10, **dadurch gekennzeichnet, dass** mindestens einer der Verankerungsköpfe in der Frontalebene zwei vordere (34, 36) und zwei hintere Abflachungen (35, 3 7) aufweist, die sich zueinander unter einem Aufweitungswinkel in der Sagittalebene erstrecken.

12. Orthese nach einem der Ansprüche 1 bis 11, **dadurch gekennzeichnet, dass** mindestens einer der Verankerungsköpfe (1, 2) mindestens eine Längsrille (38, 39) aufweist.

13. Orthese nach einem der Ansprüche 11 und 12, **dadurch gekennzeichnet, dass** mindestens einer der Verankerungsköpfe (1, 2) vier schräge Längsrillen (38, 39) mit einem gebogenen Querschnitt entlang der Längskanten jeder Abflachung (34, 35, 36, 37) aufweist.

14. Orthese nach einem der Ansprüche 1 bis 13, **dadurch gekennzeichnet, dass** die proximalen Befestigungsenden (22, 25) des tibialen Verankerungskopfs (1) und des femoralen Verankerungskopfs (2) dazu angepasst sind, nach der Implantation des Tibia-Implantats und des Femur-Implantats durch Ineinanderstecken zusammengesetzt und starr zueinander befestigt werden zu können.

15. Orthese nach Anspruch 14, **dadurch gekennzeichnet, dass** sie Mittel (26, 28) zum Sichern und Feststellen der proximalen Befestigungsenden (22, 25) zueinander aufweist.

16. Orthese nach Anspruch 15, **dadurch gekennzeichnet, dass** diese Mittel (26, 28) zum Sichern und Feststellen eine seitliche Schraube (28) aufweisen, die dazu angepasst ist, sich in die Ausbohrungen (26; 29) zu erstrecken, die im tibialen (1) und im femoralen (2) Verankerungskopf eingesetzt sind und in der frontalen Ebene ausgerichtet sind, wobei eine (29) dieser Ausbohrungen ein Innengewinde aufweist.

17. Orthese nach Anspruch 16, **dadurch gekennzeichnet, dass** die seitliche Schraube (28) ein sich verbreiterndes Ende (27) aufweist, um während des Festschraubens eine axiale Sicherung der Verankerungsköpfe (1, 2) zueinander zu bewirken.

18. Orthese nach einem der Ansprüche 1 bis 17, **dadurch gekennzeichnet, dass** einer (2) der Verankerungsköpfe (1, 2) einen Außenkegelstumpf (20) mit einem Aufbau aufweist, der dazu angepasst ist, in einen Innenkegelstumpf(21) des anderen Verankerungskopfs (1) mit entsprechendem Aufbau eingeführt zu werden, sodass sie zueinander eine konische Verbindung aufweisen.

19. Orthese nach einem der Ansprüche 1 bis 18, **dadurch gekennzeichnet, dass** der tibiale (3) und der femorale (4) Schaft einen Querschnitt ohne Rotationssymmetrie aufweisen.

20. Orthese nach einem der Ansprüche 1 bis 19, **dadurch gekennzeichnet, dass** die Abmessungen des Querschnitts des tibialen (3) und des femoralen (4) Schafts entlang dem Schaft zumindest im Wesentlichen gleichbleibend sind.

21. Chirurgischer Gerätesatz für die Kniearthrodese, **dadurch gekennzeichnet, dass** er' eine Vielzahl von vorgefertigten implantierbaren Orthesen nach einem der Ansprüche 1 bis 20 in verschiedenen Abmessungen und/oder Formen aufweist.

22. Chirurgischer Gerätesatz nach Anspruch 21, **dadurch gekennzeichnet, dass** er eine Vielzahl von tibialen Verankerungsköpfen (1), eine Vielzahl von femoralen Verankerungsköpfen (2), eine Vielzahl von tibialen Schäften (3) und eine Vielzahl von femoralen Schäften (4) in verschiedenen Abmessungen und/oder Formen aufweist.

23. Chirurgischer Gerätesatz nach einem der Ansprüche 21 oder 22, **dadurch gekennzeichnet, dass** er Modelle aufweist, die jeden Orthesenteil, den er enthält, darstellen, und die für die Auswahl der vorgefertigten implantierbaren Orthese angepasst sind, indem eine Röntgenaufnahme darüber gelegt wird.

24. Chirurgischer Gerätesatz nach einem der Ansprüche 21 bis 23, **dadurch gekennzeichnet, dass** er eine Vielzahl von Hilfsmitteln aufweist, die jeweils dazu angepasst sind, einen der Teile der vorgefertigten implantierbaren Orthesen einzusetzen.
